# EUROPEAN PATENT APPLICATION

(11) **EP 0 925 776 A2**
(43) Date of publication of application: **30.06.1999**
(21) Application number: 98123628.4
(22) Date of filing: 10.12.1998
(51) Int. Cl.: A61K 7/46, A61K 7/32

(54) **Polymer with binding capacity for organoleptic substances**

(30) Priority: 16.12.1997 EP 97122122
(71) Applicant: GIVAUDAN-ROURE (INTERNATIONAL) S.A., 1214 Vernier, Genève (CH)
(72) Inventor: Ehret, Charles, 8620 Wetzikon (CH); Lerch, Konrad, 8118 Pfaffhausen (CH); Stauch, Werner, 8400 Winterthur (CH)
(74) Representative: Buntz, Gerhard

(57) **Abstract**

The invention relates to a polymer presenting binding sites for at least one organoleptic substance, and specifically, which binding sites are at least partly molecularly imprinted with an organoleptic substance. The polymer which has the property to bind specific classes of organoleptic molecules can be used in any application where a long lasting effect of the organoleptic substance is targeted, e.g. in cosmetic products, deodorants, air refreshing products, laundry products and in fibers for fabrics. If the polymer prior has been imprinted with malodor molecules this polymer is used to reduce malodor.

## Description

The invention relates to a polymer presenting binding sites for at least one organoleptic substance, and specifically, which binding sites are at least partly molecularly imprinted with at least one organoleptic substance, which, for example, allows the slow release of odoriferous compounds. In particular, the invention relates to the production and use of these new types of polymers to trap and retain fragrance compositions, especially molecules of one specific fragrance or malodor substances or a specific malodor compound. These polymers which have the property to bind specific classes of organoleptic molecules can be used in any application where a long lasting effect of the organoleptic substance is targeted, e.g. in cosmetic products, deodorants, air refreshing products, laundry products such as detergents and fabric softeners, and in fibers for fabrics. For example, if a polymer has been imprinted with malodor molecules this polymer is used to reduce prior malodor, e.g. malodor in deodorant, cosmetic, air refreshing or laundry product applications.

A general strategy currently employed in imparting odours to consumer products is admixing of the fragrance directly to the product. The major drawback of this procedure is that most of the fragrance is lost during manufacturing, storage and use because the fragrance molecules are too volatile and/or too unstable. Often the fragrance is also lost because of lack of adhesion to the support to be perfumed, e.g. on skin, hair or fabric.

In some cases, the fragrances are microencapsulated or treated with cyclodextrins to form inclusion complexes which decrease the volatility and improve the stability. However, these methods gave often not satisfactory results or are too expensive. For example, as described in US Patent 5,382,567 a major drawback of the heretofore used cyclodextrins is their high water solubility as soon as they are used in aqueous applications.

Most materials used so far as solid phase adsorbants for above mentioned purposes act in a relatively non specific manner. Recently molecularly imprinted polymers (singular or plural form is hereafter abbreviated MIP) have received much attention to prepare receptor mimics or a kind of plastic antibodies able to recognize and bind various highly-functionalized or high molecular weight molecules, see K. Mosbach et al., The Emerging Technique of Molecular Imprinting and its Future Impact on Biotechnology, Bio/technology, February, 162-169, 1996 and M.T. Muldon et al., Plastic antibodies: molecular imprinted polymers, Chemistry & Industry, 18 March, 204-205, 1996. Thus, fragrance compounds because of their low molecular weights and their relatively low or absent functionalization seem to be unsuitable as print molecules for MIP and up to now have not been produced.

According to Mosbach et al. until now the described MIP have been applied in the following fields: chromatographic separations of pharmaceutical substances, artificial antibodies in immunoassays, use as enzyme mimics, catalysis and artificial enzymes, biosensor-like devices for structure specific detections.

According to M.T. Muldon et al. in a first step of generating a MIP the molecule to be imprinted is covalently bound to polymerizable entities or the molecule is allowed to form noncovalent interactions with polymerizable entities. These molecular assemblies are subsequently polymerized using a cross-linking agent, see Mosbach et al.. Hydrolytical extraction, or solvent extraction in case of noncovalent interactions, of the imprinted molecule furnishes rigid, insoluble, macroporous polymers which contain specific recognition and binding sites for the print molecule.

The advantages of molecularly imprinted polymers compared with biological polymers like proteins or antibodies are their easy preparation and their stability under harsh conditions, i.e. especially pH, high temperature, chemical stability and presence of solvents.

But there are several drawbacks with the MIP described up to now. Firstly the MIP known presently are suitable only for highly functionalized and non-volatile molecules and, hence, were thought not to be suitable for flavors and/or fragrances because these are too volatile, non-polar and often only poorly functionalized. According to the state of the art less than 2 mmol of imprinting product / 100 mmol of cross-linking agent are used for the preparation of the MIP.

But, secondly, the actual maximum capacity is less than 1 mg of bound target product per 1 g MIP. This capacity is too low for the use as a specific carrier for organoleptic applications, especially for applications in slow release of odoriferous molecules.

It has now surprisingly been found that, in contrast to the usual thinking of a person skilled in the art, also volatile molecules, especially flavor molecules or fragrance molecules (having at most low polarity), can be molecularly imprinted into polymers. Further an unexpected specific binding capacity could be achieved by imprinting the polymers with those molecules in higher amounts, e.g. up to 2 mmol of the odoriferous product / mmol of cross-linking agent could surprisingly be bound.

Hence, the present invention provides polymers which show specific affinities towards odiferous products and which adsorb and slowly release these molecules when used as perfuming agents in applications where long lasting effects are wished, e.g. in cosmetic products, deodorants, air refresheners, laundry products such as detergents and fabric softeners, or in fibers for fabrics.

If these polymers are e.g. imprinted with malodor molecules they can also be specifically designed to adsorb malodors for deodorization of the ambient air or to adsorb sweat malodor in deodorant applications or to absorb malodor in waste water. Examples of malodor products from sweat are steroids, e.g. androstenone (see Eigen et al., J. of the Soc. of Cosmetic Chemists, 173-185, 1991), aliphatic acids, e.g. 3-methyl-2-hexenoic acid (see Preti et al., Journal of Chemical Ecology, Vol. 17, (7), 1469-1492, 1991) and sulphur containing products (see WO 91/11988).

For the formation of the MIP according to the invention the functional monomers are polymerized with cross-linking agents in the presence of the odoriferous product(s) to be imprinted and a radical generator as catalyst, which odoriferous product(s) may be a fragrance composition, a specific fragrance, a malodor product or a specific malodor compound.

Typical functional monomers are one of acrylic acids and methacrylic acid (hereafter named as (meth)acrylic acid), vinylbenzoic acids, phenylvinyl derivatives (e.g. styrene), phenylpropenyl derivatives (e.g. eugenol, methyleugenol, eugenyl acetate) or any molecule presenting a methylene or a vinyl group, i.e. any molecule

RR'C=CH₂

where R is an aliphatic or aromatic group and R' is an aliphatic group, aromatic group or H.

Methacrylic acid and styrene are the preferred monomers.

One or more other monomers may be copolymerized with the above functional monomers. According to the invention the comonomers are specifically selected in such a way, that the rigidity of the copolymerized polymer matrix becomes improved compared with the unimproved polymer matrix. Typical comonomers of this art encompass methyl (meth)acrylate, isobornyl (meth)acrylate, vinyl acetate, vinyl halogenide).

Other functional monomers may also be used, either alone or in admixtures with the above mentioned monomers and comonomers. Functional monomers comprise for example hydroxyalkyl (meth)acrylate, (meth)acryl alkyl sulfonic acid and metal (meth)acryl alkyl sulfonates and salts of quartenarized ammonium alkyl (meth)acrylates.

Typical cross-linking agents exhibit at least two double bonds belonging to the methylene or vinyl groups. Ethylene glycol dimethacrylate, divinyl benzene or mixtures thereof being preferred. The molar ratio of cross-linking agent / functional monomer is between 1 and 10, preferably between 2 and 5.

For the preparation of the MIP the functional monomer or a mixture of some different functionalized monomers are covalently linked to or form noncovalent interactions with the imprinting products. This can be performed either in the presence of a solvent or without a solvent.

Preferred are the non-covalent interactions so that after polymerization the imprinting product(s) is/are easily removed without hydrolytic treatment in the following process step.

For example, imprinting fragrance or flavor molecules are derived from any substance or mixture of substances used in perfumery or flavor because of their olfactive properties and which present some affinities with the functional monomers, e.g. by hydrogen bonds, lipophylic or electrostatic interactions. They belong to various functional classes like alcohols, phenols, carboxylic acids, esters, carbonates, lactones, saturated and unsaturated ketones and aldehydes, aromatic coumpounds, nitrogen containing products, sulphur containing products, etc., which are known to a person skilled in the art. Some examples of fragrance or flavor products are citronellol, geraniol, phenylethyl alcohol, fixolide, cis-3-hexenol, eugenol, vanillin, substituted pyrazines and thiogeraniol.

It is quite claer, that it is not possible to give a complete list of the odoriferous products or mixture of products which can be used for imprinting in the polymers. Neither is it possible to present a complete list of malodors. But some malodors have already been mentioned above which can be used for the preparation of a MIP according to the invention.

The molar ratio of cross-linking agent / odoriferous imprinting product to is preferably between 0.5 and 50, especially between 10 and 25.

For the preparation of the polymer any inert non-aqueous organic solvent which has a good solubility for the monomers (functional monomer and the cross-linking agent) can be used, e.g. toluene, hexane or chloroform, preferably toluene. In some cases the polymerization without any solvent can be advantageous (see e.g. Example 4 as described hereafter).

The polymerization is performed with the help of standard methods known to the skilled person. Standard methods are described in the chemical literature, e.g. in M.J. Whitecombe et al., A new method for the introduction of recognition site functionally into polymers prepared by molecular imprinting: Synthesis and Characterization of polymeric receptors for cholesterol, J. Am. Chem. Soc., 117, 7105-7111, 1995. In a simple case the following standard method of free radical polymerization in solution is used: a mixture of functional monomer(s), fragrance(s), cross-linking agent(s) and solvent(s) are treated at a temperature between -10° and 70°C, preferably between 0 and 5°C, with a radical generator, e.g. α,α'-azobisisobutyronitrile (AIBN), di-tert-butyl peroxide or UV light or both. In the presence of AIBN alone a reaction temperature of 50-70°C, preferably 65°C, is needed.

The imprinted polymer can also be prepared in the presence of the molecules to be imprinted by other polymerization methods such as e.g. polyaddition, ring opening polymerization or polycondensation. Typical monomers for polycondensation are tetra-alkylsilanes and tetra-alkoxysilanes.

A preferred catalyst (initiator) as a radical generator is selected from the group consisting of diazo compounds like AIBN, peroxides like hydrogen peroxides, di-tert-butylperoxide and tert-butyl hydroperoxide. Reducing agents can advantageously be used in combination with the above mentioned peroxide. Preferred reducing agents are sodium metabisulfite and sodium formaldehyde sulfoxylate.

If required the polymer may be functionalized in order to improve its affinity of the appropriate substrates. Typical functionalized monomers comprise one of the following compounds: hydroxyalkyl (meth)acrylate, (meth)acryl alkyl sulfonic acid, metal (meth)acryl alkyl sulfonates and salts of quaternarized ammonium alkyl (meth)acrylates.

The molecularly imprinted polymers presenting (free) binding sites according to the invention are obtained by at least partly removal of the imprinting product(s) by washing with a solvent. Any solvent for the imprinting product(s) and unreacted monomer(s) may be used. Preferably the solvent is one of the group consisting of ethanol, methanol, acetic acid, or a mixture thereof, optionally with addition of water. These polymers are specifically used for binding malodor(s).

The present invention provides imprinted polymers which surprisingly are able to adsorb larger amounts of fragrant and/or flavor chemicals or perfume compositions, hereafter generally called "fragrance", which have been used for imprinting. For example, they can be loaded with about 1% to about 10% by weight of "fragrance" /polymer, preferably with about 5%. The resulting loaded polymers possess slow realease properties which make them more substantive in washing processes when used in a detergent, soap or fabric softener as when applied directly to those products. For example, when the imprinted polymer loaded with "fragrance" is used in a softener, the fragrance stays on the fabric for a much longer period as compared with a similar non-imprinted polymer.

A great advantage of the imprinted polymers according to the invention is that they can be loaded with high amounts of "fragrance". By analysis and olfactive evaluation, the "fragrance" was shown, to be released much slower from imprinted polymers in a solid state as from a similar non-imprinted polymer and that the odor is retained over a longer period of time. This aspect is crucial for any application where a slow release is important, e.g. in air freshners, cosmetics, perfuming of fabric fibers. The imprinted polymers themselves may constitute the fibers.

To a certain extent it has been found that the imprinted polymers show specificity for the imprinting molecule, e.g. phenylethanol imprinted polymer have no affinity for citronellol.

It has further been found shown that the polymer can be imprinted with a standard fragrance composition to have affinity for most of the components of the fragrance or structurally related products.

The invention also provides polymers imprinted with malodor products e.g. sweat components, smoke, tobacco smoke, to bind specifically the malodor and not the perfumes present in the application. Interesting applications of polymers which have been imprinted with sweat malodors are in the field of deodorants, air-freshners, cosmetic products. For example, it has been found that if a polymer which has been imprinted with 3-methyl-2-hexenoic acid, an important human sweat component, cf. G. Preti et al., Journal of Chemical Ecology, Vol. 17(7)1991, specifically adsorbs this sweat component out of a mixture containing a standard perfume.

The present invention is further illustrated by the following examples which are not intented to limit the effective scope of the invention.

### Examples 1

### Styrene/ethylene glycol dimethacrylate polymer imprinted with diphenyloxide for use in a softener

### Preparation of the molecularly imprinted polymer

A mixture of α,α',-azo-isobutyronitrile (AIBN, 164.0 mg, 1.0 mmol), ethyleneglycol dimethacrylate (19.82 g, 100 mmol), styrene (4.17 g,40 mmol) and diphenyloxide 1.2 g, 7.1 mmol) in toluene (40 ml) was magnetically stirred under argon while cooling on an ice bath (4°C) until complete dissolution of the AIBN. The resulting solution was transferred into a photochemical reaction vessel as a thin layer of about 5 mm thickness, cooled on an ice both and placed under an UV source (high pressure mercury lamp, 150 watts) during 17 hours. The resulting polymer was ground to small particles in a mechanical mixer. The powder was thoroughly washed with a solution of ethanol (480 ml) in deionized water (720 ml), ethanol (500 ml) and methanol(500 ml). After drying overnight under vacuum at 45°C, 14.2 g of purified, diphenyloxide free, imprinted polymer were recovered. As a control polymer exactly the same procedure was applied without diphenyloxide.

### Preparation of the softeners

The imprinted and the control polymers were both loaded with 5% w/w of diphenyloxide in 25 ml of diethyl ether and after homogenisation the solvent was completely evaporated.

The resulting loaded polymers (4.8 g, containing 0.233 g of diphenyloxid) were mixed each with a regular softener base (23.3 g, e.g. mixture Arquad 2HT from Akzo Chemie, Genapol T-150 and water in a ratio 5/1/100) and homogenized.

For the reference experiment 0.233 g diphenyloxide (1%/ softener base) was directly added to the same softener base.

### Treatment of cotton towels with a softener

The above prepared softeners were diluted with 3 liter of cold water and used for a softener treatment of newly washed cotton towels at room temperature during about 5 minutes by simulating a treatment in a washing machine. After treatment the towels have been centrifuged in a washing machine at 700 rotations/minute.
The towels have been evaluated olfactively by a panel of 10 persons and by head-space measurements in the humid state and after a 6 h and a 24 h drying phase, respectively, at room temperature. The olfactive evaluation is summerized in Table 1

**Table 1**

| | Reference | Control polymer | Imprinted polymer |
|---|---|---|---|
| humid state | ++++ | +++ | +++ |
| dry state after 6 h | + | ++ | +++ |
| dry state after 24 h | + | + | ++ |
| ++++ very strong +++ strong ++ moderate + weak | | | |

### Head-space measurements

The head-space (4-10 ml for the humid and 1000 ml for the dry towels) were absorbed on an adsorbant (Tenax), extracted with hexane and the diphenyloxide content quantified by gas chromatography (GC) analysis (30 m x 0.32 mm CP-wax 52-CB column, 60° C, 60-235° C, 10°/min.). The results are listed in Table 2.

**Table 2**

| **Concentration of diphenyloxide in the head-space (µg / l air)** | | | |
|---|---|---|---|
| | reference | control polymer | imprinted polymer |
| humid state | 108.8 | 4.62 | 0.193 |
| dry state after 6 h | 0.193 | 0.634 | 0.845 |
| dry state after 24 h | 0.193 | 0.456 | 0.566 |

### Example 2

### Styrene/ethylene glycol dimethacrylate polymer imprinted with a standard fragrance composition for use in a softener

According the procedure described in Example 1 a polymer was imprinted with 1.2 g of a standard perfume composition containing equivalent amounts of 15 volatile components as described in Table 5. The resulting polymer and corresponding control polymer have been used for the treatment of cotton towels with a softener base according to Example 1 containing a perfume loaded control polymer, a perfume loaded imprinted polymer and just the perfume as reference, respectively.
The towels have been evaluated olfactively by a panel of 10 persons and by head-space measurements as described in Example 1. The results are summerized in Tables 3 to 5.

**Table 3**

| **Olfactive evaluation** | | | |
|---|---|---|---|
| | reference | control polymer | imprinted polymer |
| humid state | ++++ | +++ | +++ |
| dry state after 6 h | + | ++ | +++ |
| dry state after 24 h | + | + | ++ |
| ++++ very strong +++ strong ++ moderate + weak | | | |

**Table 4**

| **Head-space measurements Concentration of the perfume (total of the 15 components) in the head-space (ng / l air)** | | | |
|---|---|---|---|
| | reference | control polymer | imprinted polymer |
| humid state | 79693 | 47764 | 59483 |
| dry state after 6 h | 98.8 | 1819 | 2336 |

**Table 5**

| **Concentration of the perfume components in the head-space after 6 h** | | | | |
|---|---|---|---|---|
| perfume component | reference (ng/l air) | control polymer (ng/l air) | imprinted polymer (ng/l air) | excess imprinted /control polymer (%) |
| amyl acetate | 4.7 | 853 | 840 | -1.5 |
| dimethyloctenone | 2.2 | 356 | 604 | +70 |
| eucalyptol | 0.1 | 163 | 229 | +41 |
| cyclal | 5.1 | 169 | 251 | +49 |
| linalool | 0.1 | 42.3 | 74.3 | +76 |
| ald. C12 MNA | 15.2 | 30.8 | 43.8 | +42 |
| viridine | 0.5 | 40.1 | 64.8 | +62 |
| terpineol | 4.3 | 16.7 | 26.9 | +61 |
| benzyl acetate | 3.1 | 58.8 | 86.6 | +47 |
| irisone | 4.5 | 10.1 | 11.1 | +10 |
| verdyl acetate | 2.2 | 18.4 | 27.2 | +48 |
| phenylethanol | 1.4 | 10.5 | 14.0 | +33 |
| diphenyloxide | 41.5 | 39.8 | 52.2 | +31 |
| prunolide | 2 | 6.7 | 7.0 | +5 |
| lilial | 12 | 3.9 | 4.0 | +3 |

### Example 3

### Methacrylic acid/ ethylene glycol dimethacrylate polymer imprinted with 3-methyl-2-hexenoic acid (sweat component) for deodorants

### Preparation of the molecularly imprinted polymer

A mixture of α,α'-azo-isobutyronitrile (AIBN, 82.1 mg, 0.5 mmol), ethyleneglycol dimethacrylate (9.91 g, 50 mmol), methacrylic acid (1.72 g, 20 mmol) and the sweat component 3-methyl-2-hexenoic acid (256.3 mg, 2 mmol) in toluene (20 ml) was magnetically stirred under argon while cooling in an ice bath until complete dissolution of the AIBN. The resulting solution was transfered into a photochemical reaction vessel as a thin layer of about 5 mm thickness, cooled on ice (4° C) and placed under an UV source (high pressure mercury lamp, 150 watts) during 17 hours. The resulting polymer was ground to small particles in a mechanical mixer. The polymeric powder was thoroughly washed resp. with a mixture of ethanol (240 ml), acetic acid (120 ml) and deionized water (240 ml), a mixture of ethanol (200 ml) and acetic acid (50ml) and finally with pure methanol (250 ml). After drying overnight under vacuum at 45°C, 8.21 g of purified, with 3-methyl-2-hexenoic acid imprinted polymer were recovered. As a control exactly the same procedure was applied without the methyl hexenoic acid.

The imprinted and the control polymers (100 mg) were each loaded with 1 ml of a diethyl-ether solution of 3-methyl-2-hexenoic acid (1 g/l), 1 ml of a diethylether solution of the standard perfume as described in Example 2, (1 g/l) and 50 µl of water. After homogenisation the ether is evaporated by gentle heating at about 40 °C during 10 min. After 3 hours resting at room temperature in air the samples were olfactively evaluated:

In a panel of 9 persons 8 persons found that the imprinted sample has a much more pleasant, cleaner, less sweaty odour compared to the nonimprinted control.

### Example 4

### Methacrylic acid/ ethylene glycol dimethacrylate polymer imprinted with citronellol for use in air freshners, cosmetic products or fabric fibers

### Preparation of the molecularly imprinted polymer without solvent

A mixture of α,α'-azo-isobutyronitrile (AIBN, 65.7 mg, 0.4 mmol), ethyleneglycol dimethacrylate (9.91 g, 50 mmol), methacrylic acid (861.0 mg, 10 mmol) and citronellol (12.9 g, 83mmol) was magnetically stirred under argon while cooling in an ice bath until complete dissolution of the AIBN. The resulting solution was transfered into a photochemical reaction vessel as a thin layer of about 5 mm thickness, cooled on ice (4° C) and placed under an UV source (high pressure mercury lamp, 150 watts) during 16 hours. The resulting polymer was ground to small particles in a mechanical mixer. The polymeric powder was thoroughly washed with a mixture of ethanol (240 ml), acetic acid (120 ml) and deionized water (240 ml), a mixture of ethanol (200 ml) and acetic acid (50ml) and finally with pure methanol (250 ml). After drying overnight under vacuum at 45°C, 8.21 g of purified, with citronellol imprinted polymer were recovered.

For a reference polymer imprinted with less citronellol the same procedure was applied with additional 15 ml of toluene and only 195.9 mg (1.25 mmol of citronellol).

As a control exactly the same procedure was applied without citronellol and with 15 ml of toluene.

50 mg of each of the three polymers were suspended during 18 h in 5 ml of a 70% methanol solution containing 0.025 mg citronellol/ml. After centrifugation the amount of adsorbed citronellol was evaluated by GC analysis. The amounts of citronellol (%/polymer) adsorbed on the control polymer and on the imprinted polymers prepared with an without solvent are as presented in Table 6.

**Table 6**

| Control (no citronellol) | Reference (citronellol and solvent) | polymer with citronellol as solvent |
|---|---|---|
| 0.59 % | 0.95 % | 1.32 % |

The adsorption capacity for citronellol increases with the amount of citronellol used for imprinting.

### Example 5

### Methacrylic acid/ ethylene glycol dimethacrylate polymer imprinted with citronellol for use in airfresners, cosmetic products or fabric fibers

### Preparation of the molecularly imprinted polymer in the presence of toluene

A mixture of α,α'-azo-isobutyronitrlie (AIBN, 82.1 mg, 0.5 mmol), ethyleneglycol dimethacrylate (9.91 g, 50 mmol), methacrylic acid (1.72 g, 20 mmol) and citronellol (2.476 g, 15.8 mmol) in toluene (10 ml) was magnetically stirred under argon while cooling in an ice bath until complete dissolution of the AIBN. The resulting solution was transferred into a photochemical reaction vessel as a thin layer of about 5 mm thickness, cooled on ice (4° C) and placed under an UV source (high pressure mercury lamp, 150 watts) during 18 hours. The resulting polymer was ground to small particles in a mechanical mixer. The polymeric powder was thoroughly washed with a mixture of ethanol (240 ml), acetic acid (120 ml) and deionized water (240 ml), a mixture of ethanol (200 ml) and acetic acid (50ml) and finally with pure methanol (250 ml). After drying overnight under vacuum at 45°C, 8.3 g of purified, with citronellol imprinted polymer were recovered. As a reference exactly the same procedure was applied without the citronellol.

### Evaluation by adsorption in the solid phase

The imprinted polymer and the reference polymer were loaded with citronellol (50 mg/g as described in Example 1) and compared by GC analysis of methanol extracts and olfactive evaluation over a period of 14 days. The results are shown in Table 7.

**Table 7**

| (mg/100 mg polymer) | | | | | |
|---|---|---|---|---|---|
| | day 0 | day 1 | day 3 | day 7 | day 14 |
| imprinted polymer | 3.5 | 3.3 | 4.0 | 3.8 | 3.6 |
| reference polymer | 3.9 | 3.0 | 3.2 | 3.2 | 3.3 |

A panel of 11 persons found unanimously that the imprinted material relative to olfactive properties was significantly stronger after 18 days.

### Example 6

### Mixed styrene/methacrylic acid/ ethylene glycol dimethacrylate polymer imprinted with 2-phenylethanol for use in air freshners or fabric fibers

### Preparation of the molecularly imprinted polymer

A mixture of α,α'-azo-isobutyronitrile (AIBN, 82.1 mg, 0.5 mmol), ethyleneglycol dimethacrylate (9.91 g, 50 mmol), methacrylic acid (861.0 mg, 10 mmol), styrene (1.04 g, 10 mmol) and 2-phenylethanol (244.3 mg, 2 mmol) in toluene (20 ml) was magnetically stirred under argon while cooling in an ice bath until complete dissolution of the AIBN. The resulting solution was transfered into a photochemical reaction vessel as a thin layer of about 5 mm thickness, cooled on ice (4° C) and placed under an UV source (high pressure mercury lamp, 150 watts) during 17 hours. The resulting polymer was ground to small particles in a mechanical mixer. The polymeric powder was thoroughly washed resp. with a mixture of ethanol (240 ml), acetic acid (120 ml) and deionised water (240 ml), a mixture of ethanol (200 ml) and acetic acid (50ml) and finally with pure methanol (250 ml). After drying over- night under vaccuum at 45°C, 6.45 g of purified, with phenylethanol imprinted polymer were recovered. As a reference exactly the same procedure was applied without phenylethanol.

### Evaluation by absorption in the solid phase

Imprinted polymer and reference polymer samples were loaded with 50 mg/g phenylethanol, 50 mg/g citronellol and with the standard perfume composition (50 mg/g, 15 components, see Example 2) and evaluated after 14 days at room temperature by GC (Solid Phase Micro Extraction (SPME), Supelco). The results are summerized in Table 8.

**Table 8**

| **GC/SPME head-space evaluation of loaded polymers imprinted with phenylethanol** | |
|---|---|
| **Sample** | **area counts** |
| imprinted loaded with phenylethanol | 96410 |
| control loaded with phenylethanol | 47577 |
| imprinted loaded with citronellol | 45262 |
| control loaded with citronellol | 47262 |
| imprinted loaded with perfume* | 11644 |
| reference loaded with perfume* | 9252 |

| | |
|---|---|
| *perfume as described in Example 2 | |

The results show that the phenylethanol imprinted polymer show high specificity for phenylethanol, specificity for the perfume and no specificity for citronellol.

## Claims

1. Polymer presenting binding sites for at least one organoleptic substance.

2. The polymer of claim 1 characterized in that the binding sites are molecularly imprinted with at least one organoleptic substance.

3. The polymer of claim 1 or 2, whereby the organoleptic substance is selected from the group consisting of odoriferous compounds, flavor compounds and malodor compounds.

4. The polymer of claim 3, whereby the odoriferous compounds are selected from the group consisting of alcohols, phenols, carboxylic acids, esters, carbonates, lactones, saturated or unsaturated ketones, aldehydes, aromatic compounds, nitrogen containing compounds or any mixture of such compounds.

5. The polymer of claim 3, whereby the flavor compounds are selected from the group consisting of of alcohols, phenols, carboxylic acids, esters, lactones, saturated or unsaturated ketones, aldehydes, aromatic compounds, nitrogen containing compounds or any mixture of such compounds.

6. The polymer of claim 3, whereby the malodor compounds are selected from compounds existing in human sweat, especially steroids, aliphatic acids and sulphur containing compounds.

7. The polymer according claim 6, wherein the malodor compound is 3-methyl-2-hexenoic acid.

8. The polymer of any one of the claims 1 to 6, whereby the odorifereous or flavor compound(s) is/are slowly released.

9. The polymer according to claim 8, wherein the slow release of the odorifereous compound(s) is/are effected by evaporation from the solid state or by release in an aqueous or an organic solvent suspension.

10. The polymer of any one of the claims 1 to 9 whereby the binding sites are partly imprinted with at least one odorifereous compound and are partly able to bind molodor compounds.

11. The polymer of any one of the claims 2 to 9 produced by a polymerization of at least one functional monomer and at least one cross-linking agent in the presence of at least one organoleptic substance.

12. The polymer of claim 11, whereby the imprinted organoleptic substance(s) is/are at least partly removed by washing the polymer in a solvent for the organoleptic substance(s).

13. The polymer according to claim 12, whereby the solvent is selected from the group consisting of ethanol, methanol, acetic acid and mixtures thereof, optionally with addition of water.

14. The polymer of claim 12, whereby the organoleptic substance(s) is/are (a) malodor compound(s).

15. The polymer of claim 12 characterized in that two types of binding sites are present, whereby one type is imprinted with odoriferous compound(s) and the other type has been imprinted with at least one malodor compound which malodor compound(s) thereafter has/have been dissolved by washing with an appropriate solvent.

16. The polymer according to claims 11, wherein the functional monomer exhibits a group with a double bond, preferably a methylene or a vinyl group.

17. The polymer according to claim 16, wherein the functional monomer is at least one of the group consisting of acrylic acid, methacrylic acid, styrene and phenylpropenyl derivatives.

18. The polymer according to claim 11, wherein the cross-linking agent exhibits at least two groups with double bonds, preferably two methylene or vinyl groups.

19. The polymer according to claim 18, wherein the cross-linking agent is ethylene glycol dimethyl acrylate, divinyl benzene or a mixture thereof.

20. The polymer according to any one of the claims 11 and 16 to 19, wherein the molar ratio of cross-linking agent/functional monomer is between 1 and 10, preferably between 2 and 5.

21. The polymer according to any one of the claims 11 and 16 to 20, wherein the molar ratio of cross-linking agent/imprinting odorifereous substance is between 0.5 and 50, preferably between 10 and 25.

22. The polymer according to any one of the claims 11 and 16 to 21, whereby the polymerization has been effected in the presence of a catalyst as a radical generator and/or UV light.

23. The polymer according to any one of the claims 11 and 16 to 22, whereby the polymer has been obtained by polyaddition, ring opening polymerization or polycondensation.

24. The polymer according to any one of the claims 1 to 23, used for perfuming cosmetic products, deodorants, air-refreshing products, laundry products, especially detergents or fabric softeners, and fabric fibers.

25. The polymer according to any one of the claims 10 or 12 to 15, whereby the imprinting of malodor compound(s) is/are used for reduction of malodor in deodorant, cosmetic, air-refreshing or laundry applications.

26. The polymer according to any one of the claims 1 to 25, whereby the imprinted substance(s) is present in about 1 to 10% by weight.

27. A process for the manufacture of the molecularly imprinted polymers according to any one of the claims 2 to 26 comprising polymerization of a mixture of functional monomer(s) and at least one cross-linking agent in the presence of at least one organoleptic substance followed by removal of the organoleptic substance(s).

28. A process for the manufacture of the molecularly imprinted polymers according to claim 1 comprising polymerization of a mixture of functional monomer(s) and at least one cross-linking agent in the presence of at least one organoleptic substance followed by removal of the organoleptic substance(s).

29. The process according to claim 27 or 28 whereby the removal is performed by washing with a solvent for the imprinted organoleptic substance(s).
